# EUROPEAN PATENT SPECIFICATION

(11) **EP 0 575 841 B1**
(45) Date of publication and mention of the grant of the patent: **28.08.1996**
(21) Application number: 93109425.4
(22) Date of filing: 12.06.1993
(51) Int. Cl.: A61K 31/00, A61K 31/47, A61K 31/56, A61K 31/60, A61K 33/06

(54) **Composition containing dibucaine hydrochloride, a salicylate, calcium bromide and an antiphlopstic steroid for the treatment of pain**
Zusammensetzung enthaltend Dibucaine-Hydrochlorid, ein Salicylat, Calciumbromid und ein antiphlogistisches steroid zur Schmerzbekämpfung
Composition contenant le hydrochlorure de dibucaine, un salicylate, le bromure de calcium et un stéroid antiphlogistique pour le traitement de la douleur

(30) Priority: 16.06.1992 JP 156961/92
(43) Date of publication of application: 29.12.1993
(73) Proprietor: VITACAIN PHARMACEUTICAL CO., Ltd., Moriguchi-shi, Osaka 570 (JP)
(72) Inventor: Hyodo, Masayoshi, Takatsuki-shi, Osaka 569 (JP); Akiyoshi, Masataka, Ashiya-shi, Hyogo 659 (JP)
(74) Representative: von Kreisler, Alek, Dipl.-Chem.

(56) References cited:
- PHARMACOMETRICS (JAPAN), vol. 20, no. 4, 1980, pages 693-701; TSUTOMU KAMEYAMA ET AL.: 'Dibucaine hydrochloride, sodium salixylate and calcium bromide'
- J. INDIAN MED. ASSOC, vol. 82, no. 11, 1984, pages 397-399; SAMIR K. GUPTA ET AL.: 'Tietze's Syndrome'

## Description

### FIELD OF THE INVENTION

The present invention relates to injectable compositions for treating pain, and a novel use of antiphlogistic steroids.

### BACKGROUND OF THE INVENTION

Conventionally, pain caused by rheumatic diseases and locomotorium diseases has been treated with nonsteroidal antiinflammatory, local anesthetic and antispasmodic which are generally referred to as analgesics in a wide sense, and with vasoconstriction, vasodilator and muscular relaxant having other actions as well, which may be used in combination. This treatment using plural medicaments is based on the pain developing mechanisms, and the mechanism of action of medicaments. For example, a nonsteroidal antiinflammatory exhibits etiotropic effects by inhibiting synthesis of the pain-producing substance prostaglandins, while a local anesthetic exhibits symptomatic effects by blocking transmission of neurostimulation from a local pain source to the center, and the combination of these medicaments aims at synergistic effects afforded by their different actions displayed at different sites. An exemplary of the medicaments formulated for achieving such synergistic effects is Neo Vitacain injection ® (VITACAIN PHARMACEUTICAL CO., LTD., Japan), which is a local injection containing dibucaine hydrochloride, sodium salicylate, and calcium bromide as active ingredients and having a formulation shown below. This injection is markedly effective as an analgesic for use in a pain treatment of head neuralgia, muscle pain, rheumatism and, low backache (for discussion of these synergistic effects see Pharmacometics 20(4), 693-701 (1980)). Usefulness of Neo Vitacain injection® and its analogous therapeutic compositions for the treatment of pain will be greatly enhanced if the effect thereof could be improved more.

| Formulation of Neo Vitacain injection® (100 ml): | |
|---|---|
| Dibucaine hydrochloride | 100 mg |
| Sodium salicylate | 300 mg |
| Calcium bromide | 200 mg |
| Thiamine hydrochloride | 200 mg |
| Pyridoxin hydrochloride | 100 mg |
| Calcium pantothenate | 100 mg |

### SUMMARY OF THE INVENTION

An object of the present invention is to provide novel pharmaceutical compositions effective for algetic diseases.

Another object of the present invention is to provide a novel use of antiphlogistic steroids as contained in a composition for reinforcing pain relief action, which is used with other analgesic such as Neo Vitacain injection® for the treatment of pain.

The present inventors have conducted intensive studies with the aim of achieving the above-mentioned purposes, and found that a composition obtained by adding antiphlogistic steroid(s) to a composition containing dibucaine, a pharmaceutically acceptable salt of salicylic acid, and calcium bromide has a pain relief effect which is superior to that of the conventional agents for treating pain, and that said composition is extremely useful for the treatment of pain. Moreover, the present inventors have found that the treatment using a composition containing dibucaine, a pharmaceutically acceptable salt of salicylic acid, calcium bromides and antiphlogistic steroid(s) is an extremely effective method for reducing pain. Still further, the present inventors have found that an antiphlogistic steroid can reinforce pain relief effect of a composition containing dibucaine, a pharmaceutically acceptable salt of salicylic acid, and calcium bromide. With these findings, the present inventors have completed the present invention.

That is, the present invention is:
1. An injectable composition for treating pain, which contains dibucaine, a pharmaceutically acceptable salt of salicylic acid, calcium bromide, and antiphlogistic steroid(s);
2. Use of a composition containing dibucaine, a pharmaceutically acceptable salt of salicylic acid, calcium bromide and an antiphlogistic steroid, for the production of an injection for treating pain.
3. A composition containing antiphlogistic steroid(s) and pharmaceutically acceptable carriers for reinforcing pain relief action of a composition containing dibucaine, a pharmaceutically acceptable salt of salicylic acid, and calcium bromide; and
4. Use of antiphlogistic steroid(s) as contained in a composition for reinforcing pain relief action of a composition containing dibucaine, a pharmaceutically acceptable salt of salicylic acid, and calcium bromide.

### DETAILED DESCRIPTION OF THE INVENTION

The pain here particularly means pain at a local region or site, and includes myofascial pain, circumomarthritis, symptomatic neuralgia, and transdermal, muscular and articular pain accompanying locomotorium diseases. Specific examples include occipital neuralgia, temporal neuralgia, muscle pain, neuralgia, chronic rheumatism, low backache, backache, pain caused by autonomic nerve sensitivity, migraine, trigeminal pain, frozen shoulder, and pain caused by disorders of autonomic nerve (e.g. algetic insomnia, menopausal neuralgia).

The treatment of pain in this specification means treatments given for the purpose of removing or alleviating the above-mentioned pains.

The dibucaine to be used in the present invention is normally in the form of a salt, with preference given to dibucaine hydrochloride.

The antiphlogistic steroid to be used in the pharmaceutical composition of the present invention may be any antiphlogistic steroid as long as it can be used as an injection, and examples thereof include dexamethasone, betamethasone, methylprednisolone, prednisolone, and salts thereof. These antiphlogistic steroids are normally used in the form of pharmaceutically acceptable salts, and are exemplified by dexamethasone acetate, dexamethasone palmitate, dexamethasone sodium phosphate, dexamethasone sodium metasulfobenzoate, betamethasone acetate, betamethasone sodium phosphate, prednisolone acetate, prednisolone butylacetate, prednisolone sodium succinate, methylprednisolone acetate and methylprednisolone sodium succinate, with preference given to dexamethasone sodium phosphate, betamethasone sodium phosphate, and methylprednisolone acetate.

The pharmaceutically acceptable salt of salicylic acid to be used in the present invention is exemplified by alkali metal salts, and preferred is sodium salt.

The composition for treating pain of the present invention contains each component at a proportion of 100-1000 parts by weight, preferably 200-500 parts by weight of a pharmaceutically acceptable salt of salicylic acid, and 50-500 parts by weight, preferably 100-300 parts by weight of calcium bromide per 100 parts by weight of dibucaine. The proportion of the antiphlogistic steroid varies depending on the kind thereof, and dexamethasone and betamethasone are normally contained in a proportion of 4-80 parts by weight, preferably 6-40 parts by weight, and prednisolone and methylprednisolone are normally contained in a proportion of 80-2000 parts by weight, preferably 100-1000 parts by weight per 100 parts by weight of dibucaine.

In Table 1, the amounts of various antiphlogistic steroids to be added to 5 ml of, for example, Neo Vitacain injection® are detailedly shown. The amounts shown here are on an antiphlogistic steroid basis, and in practice, they are recalculated into the salt amounts to be preferably used.

**Table 1**

| Amounts of various steroids to be added to 5 ml of Neo Vitacain | | |
|---|---|---|
| Steroid to be added | normal amount (mg) | preferable amount (mg) |
| Dexamethasone | 0.2 - 4.0 | 0.3 - 2.0 |
| Betamethasone | 0.2 - 4.0 | 0.3 - 2.0 |
| Methylprednisolone | 4.0 - 80.0 | 5.0 - 40.0 |
| Prednisolone | 5.0 -100.0 | 5.0 - 50.0 |

The composition of the present invention may contain additives normally accepted from the aspect of the formulation of pharmaceutical preparations. For example, carriers, stabilizers (e.g. creatinine), solubilizers (e.g. glycerin), suspending agents (e.g. carboxymethylcellulose), buffers (e.g. citric acid, sodium hydrogencarbonate), emulsifiers (e.g. fatty acid monoglyceride, sorbitan fatty acid ester and polyoxyethylene lauryl ether), antiseptics (e.g. methyl p-oxybenzoate and propyl p-oxybenzoate) and antioxidants (e.g. t-butylhydroxyanisole) may be added to liquid compositions, and excipients may be further added to powder compositions.

In the present invention, the aforementioned compositions are preferably Injected into the location site of pain, and for this reason, the compositions of the present invention for the treatment of pain may take any form as long as they can be prepared into injections when in use. Examples thereof include aqueous solution, suspension, emulsion, and powders to be dissolved, suspended or emulsified when in use. They may be prepared by conventional methods. The pH of the aqueous solution, suspension, and emulsion is preferably within the range of from pH 4 to 7, particularly preferably at about pH 6, in which a long-term storage can be attained despite the instability of dexamethasone phosphate in the acidic range and instability of dibucaine hydrochloride in the alkali range, and pH of the preparations is adjusted to said range by conventional methods. Also, the powder compositions may be prepared in such a manner as adjusts their pH to be in the range of from 4 to 7, preferably about 6 upon dissolution in sterilized water or sterilized physiological saline.

The composition for the treatment of pain, and the composition for reinforcing pain relief action of the present invention are administered to mammals such as human, dog, cow, horse and cat, particularly to human.

The composition for treating pain and composition for reinforcing pain relief action of the present invention are injected locally, such as into muscle, peritenon and articular cavity.

While the dose of the composition for treating pain of the present invention varies depending on age of patients, symptom, administration site and drugs to be used, taking a therapeutic composition of the above-described formulation wherein antiphlogistic steroid(s) is(are) added to 5 ml of Neo Vitacain injection® in an amount given in Table 1, as an example, it is normally 0.1-25 ml, preferably 0.5-5 ml per site, namely, 0.1-25 mg, preferably 0.5-5 mg of dibucaine hydrochloride, 0.1-250 mg, preferably 1-25 mg of sodium salicylate, 0.05-125 mg, preferably 0.5-15 mg of calcium bromide, to which 0.004-500 mg of antiphlogistic steroid(s) may be added, wherein 0.004-20 mg, preferably 0.03-2 mg of dexamethasone and/or betamethasone, and/or 0.08-500 mg, preferably 0.5-50 mg of methylprednisolone and/or prednisolone is(are) used as the antiphlogistic steroid(s)

Since the use of the composition of the present invention for treating pain permits long-lasting and potent pain treating effects, intermittent administration can be performed. While administration frequency differs depending on age of patients, symptom and pain location, administration of once or twice a week, or 1 to 4 times in 2 weeks can exert sufficient effects.

The use of the composition of the present invention for treating pain affords not only superior pain relief effect but also reduced pain at the time of the needle puncture and insertion of a drug.

In the present invention, the composition for reinforcing pain relief action is a general concept embracing those having an action of potentiating pain relief action when added to a compound or composition having pain relief action or when used independently but together with said compound or composition. In the present invention, it means a composition containing, as an active ingredient, antiphlogistic steroid which is capable of additionally reinforcing pain reducing effect possessed by a composition containing dibucaine, a pharmaceutically acceptable salt of salicylic acid and calcium bromide, when added thereto or used independently but together with said composition.

The composition for reinforcing pain relief action of the present invention may be in the form of injectable solutions, suspensions or emulsions, or powders which can be dissolved, suspended or emulsified when in use as an injection.

The aforementioned additives for the composition for treating pain may be also added to the composition for reinforcing pain relief action.

The use of the composition for reinforcing pain relief action may be performed by adding same to an injectable composition containing dibucaine, a pharmaceutically acceptable salt of salicylic acid, and calcium bromide, particularly Neo Vitacain injection®, or by an independent use thereof together with a composition containing dibucaine, a pharmaceutically acceptable salt of salicylic acid, and calcium bromide when it is used for the treatment of pain.

The composition for reinforcing pain relief action of the present invention is preferably used in admixture with an injectable composition containing dibucaine, a pharmaceutically acceptable salt of salicylic acid, and calcium bromide. The composition for reinforcing pain relief action is added to said composition containing dibucaine, a pharmaceutically acceptable salt of salicylic acid, and calcium bromide in the same amount as that in the aforementioned composition for treating pain, on an antiphlogistic steroid basis. That is, the composition for reinforcing pain relief action is added in an amount of 0.25-800 parts by weight per 100 parts by weight of the total amount of dibucaine, a pharmaceutically acceptable salt of salicylic acid, and calcium bromide, on an antiphlogistic steroid basis.

Each component and the composition containing dibucaine, a pharmaceutically acceptable salt of salicylic acid, and calcium bromide to be used in the present invention have been conventionally used as pharmaceuticals, and safety thereof in the above-mentioned administration amounts has been already established. For example, acute toxicity of Neo Vitacain injection® is as shown in LD₅₀ of 34 ml/kg, and addition thereto or independent use together therewith of antiphlogistic steroid in an amount mentioned above did not result in significant increase of toxicity.

| Example 1 (Injection formulation, 100 ml) | |
|---|---|
| Dibucaine hydrochloride | 100 mg |
| Sodium salicylate | 300 mg |
| Calcium bromide | 200 mg |
| Thiamine hydrochloride | 200 mg |
| Pyridoxin hydrochloride | 100 mg |
| Calcium pantothenate | 100 mg |
| Dexamethasone sodium phosphate | 20 mg |

| Example 2 (Injection formulation, 100 ml) | |
|---|---|
| Dibucaine hydrochloride | 100 mg |
| Sodium salicylate | 300 mg |
| Calcium bromide | 200 mg |
| Betamethasone sodium phosphate | 20 mg |

| Example 3 (Injection formulation, 100 ml) | |
|---|---|
| Betamethasone sodium phosphate | 200 mg |
| Sodium sulphite | 30 mg |
| p-Oxybenzoic acid | 75 mg |
| Creatinine | 400 mg |

| Example 4 (Composition for reinforcing pain relief action, Injection formulation, 100 ml) | |
|---|---|
| Methylprednisolone acetate | 2 g |
| Polyethylene glycol | 3 g |

The pain relief action of the composition for treating pain of the present invention, and the reinforcing effect of the composition for reinforcing pain relief action of the present invention on the agents for treating pain are hereinbelow described in detail.

### Experiment 1

To a patient (male, age 63) who had been suffering from myofascial low backache for 2 months was administered the following medicament once a week, and the degree of pain before administration every week was compared.

In evaluating the pain, the initial pain was taken as 10, and the absence of pain was taken as 0, which is the same in the following experiments.

### Medicament administered

- A :: Neo Vitacain 3 ml
- B :: Neo Vitacain 3 ml + Dexamethasone sodium phosphate 0.5 mg

### Administration site

- Medicament A :: left lumbar region
- Medicament B :: right lumbar region

The results are summarized in Table 2, in which it is shown that addition of an antiphlogistic steroid to Neo Vitacain resulted in earlier removal of the pain than the sole use of Neo Vitacain, thereby indicating remarkable potentiation of the effect of pain treatment.

**Table 2**

| | before 1st administration | before 2nd administration | before 3rd administration | before 4th administration | before 5th administration |
|---|---|---|---|---|---|
| Neo Vitacain | 10 | 6 | 4 | 3 | 1 |
| Neo Vitacain + steroid | 10 | 4 | 0 | - | - |

### Experiment 2

To a patient (female, age 27) who had been suffering from tenontothecitis of both thumbs for 3 months was administered the following medicament once a week, and the effect was evaluated as in Experiment 1.

### Medicament administered

- A :: Neo Vitacain 0.5 ml
- B :: Neo Vitacain 0.5 ml + Dexamethasone sodium phosphate 0.1 mg

### Administration site

- Medicament A :: right thumb
- Medicament B :: left thumb

The results are summarized in Table 3, in which it is shown that addition of an antiphlogistic steroid to Neo Vitacain resulted in earlier removal of the pain than the sole use of Neo Vitacain, thereby indicating remarkable potentiation of the effect of pain treatment.

**Table 3**

| | before 1st administration | before 2nd administration | before 3rd administration | before 4th administration |
|---|---|---|---|---|
| Neo Vitacain | 10 | 7 | 5 | 3 |
| Neo Vitacain + steroid | 10 | 3 | 0 | - |

### Experiment 3

To a patient (male, age 46) who had been suffering from myofascial pain (*tenchu* syndrome) at the nuchal region for 1 month was administered the following medicament particularly at the *tenchu* part of the right and left nuchal regions, and the effects were evaluated as in Example 1.

### Medicament administered

- A :: Neo Vitacain 2 ml
- B :: Neo Vitacain 2 ml + Methylprednisolone acetate 4 mg

### Administration site

- Medicament A :: left nuchal region
- Medicament B :: right nuchal region

The results are summarized in Table 4, in which it is shown that addition of an antiphlogistic steroid to Neo Vitacain resulted in earlier removal of the pain than the sole use of Neo Vitacain, thereby indicating remarkable potentiation of the effect of pain treatment.

**Table 4**

| | before 1st administration | before 2nd administration | before 3rd administration | before 4th administration | before 5th administration |
|---|---|---|---|---|---|
| Neo Vitacain | 10 | 7 | 5 | 4 | 4 |
| Neo Vitacain + steroid | 10 | 4 | 2 | 2 | 0 |

## Claims

1. An injectable composition for treating pain, which contains dibucaine, a pharmaceutically acceptable salt of salicylic acid, calcium bromide and antiphlogistic steroid(s).

2. A composition for treating pain according to Claim 1, which is a liquid having a pH in the range of from 4 to 7.

3. A composition for treating pain according to Claim 1 or Claim 2, which contains 100 parts by weight of dibucaine, 100-1000 parts by weight of a pharmaceutically acceptable salt of salicylic acid, 50-500 parts by weight of calcium bromide, and 4-2000 parts by weight of antiphlogistic steroid(s).

4. A composition for treating pain according to Claim 1, which contains 0.1-25 mg of dibucaine hydrochloride, 0.1-250 mg of a pharmaceutically acceptable salt of salicylic acid, 0.05-125 mg of calcium bromide, and 0.004-500 mg of antiphlogistic steroid(s) per administration at one site.

5. A composition for treating pain according to any one of Claims 1 to 4, wherein the antiphlogistic steroid is at least one member selected from the group consisting of dexamethasone, betamethasone, methylprednisolone, prednisolone and their pharmaceutically acceptable salts.

6. Use of a composition containing dibucaine, a pharmaceutically acceptable salt of salicylic acid, calcium bromide and an antiphlogistic steroid, for the production of an injection for treating pain.

7. The use according to Claim 6, wherein the composition contains each component in a proportion of 100 parts by weight of dibucaine, 100-1000 parts by weight of a pharmaceutically acceptable salt of salicylic acid, 50-500 parts by weight of calcium bromide, and 4-2000 parts by weight of antiphlogistic steroid(s).

8. The use according to Claim 6 or Claim 7, wherein the composition is a liquid having a pH in the range of from 4 to 7.

9. The use of Claim 6, wherein the injection is administered once or twice a week.

10. The use of Claim 6, wherein the injection is administered 1-4 time(s) a week.

11. The use of Claim 9 or Claim 10, wherein the composition contains 0.1-25 mg of dibucaine hydrochloride, 0.1-250 mg of the pharmaceutically acceptable salt of salicylic acid, 0.05-125 mg of calcium bromide and 0.004-500 mg of the antiphlogistic steroid per dose.

12. A composition for reinforcing pain relief action of a composition containing dibucaine, a pharmaceutically acceptable salt of salicylic acid, and calcium bromide, which contains antiphlogistic steroid as an active ingredient.

13. A composition according to Claim 12, which is for reinforcing pain relief action of a composition containing 100 parts by weight of dibucaine, 100-1000 parts by weight of a pharmaceutically acceptable salt of salicylic acid, and 50-500 parts by weight of calcium bromide.

14. A composition for reinforcing pain relief action according to Claim 12 or Claim 13, wherein the antiphlogistic steroid is at least one member selected from the group consisting of dexamethasone, betamethasone, methylprednisolone, prednisolone and their pharmaceutically acceptable salts.

15. Use of an antiphlogistic steroid for the production of a composition having a potentiated pain relief action, which contains, as an active ingredient, the antiphlogistic steroid, the composition being used for treating pain along with a composition containing dibucaine, a pharmaceutically acceptable salt of salicylic acid and calcium bromide.

16. The use of Claim 15, wherein the composition having a potentiated pain relief action is used by being added to an injectable composition containing dibucaine, a pharmaceutically acceptable salt of salicylic acid and calcium bromide.

17. The use of Claim 15, wherein the composition having a potentiated pain relief action is used independently but together with a composition containing dibucaine, a pharmaceutically acceptable salt of salicylic acid and calcium bromide.

18. The use according to any one of Claims 15 to 17, wherein the antiphlogistic steroid is at least one member selected from the group consisting of dexamethasone, betamethasone, methylprednisolone, prednisolone and their pharmaceutically acceptable salts.

19. The use according to any one of Claims 15 to 17, wherein the antiphlogistic steroid is contained, in a proportion of 0.25-800 parts by weight per 100 parts by weight of the total amount of dibucaine, a pharmaceutically acceptable salt of salicylic acid, and calcium bromide.

## Patentansprüche

1. Injizierbare Zusammensetzung zum Behandeln von Schmerzen, umfassend Dibucain, ein pharmazeutisch annehmbares Salz von Salicylsäure, Calciumbromid und (ein) entzündungshemmende(s) Steroid(e).

2. Zusammensetzung zum Behandeln von Schmerzen nach Anspruch 1, die eine Flüssigkeit mit einem pH-Wert im Bereich von 4 bis 7 ist.

3. Zusammensetzung zum Behandeln von Schmerzen nach Anspruch 1 oder Anspruch 2, die 100 Gew.-Teile Dibucain, 100 bis 1000 Gew.-Teile eines pharmazeutisch annehmbaren Salzes von Salicylsäure, 50 bis 500 Gew.-Teile Calciumbromid und 4 bis 2000 Gew.-Teile (eines) entzündungshemmende(n) Steroide (Steroids) enthält.

4. Zusammensetzung zum Behandeln von Schmerzen nach Anspruch 1, die 0,1 bis 25 mg Dibucain, 0,1 bis 250 mg eines pharmazeutisch annehmbaren Salzes von Salicylsäure, 0,05 bis 125 mg Calciumbromid und 0,004 bis 500 mg (eines) entzündungshemmende(n) Steroide (Steroids) pro Verabreichung an einer Stelle enthält.

5. Zusammensetzung zum Behandeln von Schmerzen nach einem der Ansprüche 1 bis 4, wobei das entzündungshemmende Steroid wenigstes ein Element ist, das aus der aus Dexamethason, Betamethason, Methylprednisolon, Prednisolon und deren pharmazeutisch annehmbaren Salzen bestehenden Gruppe ausgewählt ist.

6. Verwendung einer Zusammensetzung, die Dibucain, ein pharmazeutisch annehmbares Salz von Salicylsäure, Calciumbromid und ein entzündungshemmendes Steroid enthält, zur Herstellung einer Injektion zur Behandlung von Schmerzen.

7. Verwendung nach Anspruch 6, wobei die Zusammensetzung jede Komponente mit einem Anteil von 100 Gew.-Teilen Dibucain, 100 bis 1000 Gew.-Teilen eines pharmazeutisch annehmbaren Salzes von Salicylsäure, 50 bis 500 Gew.-Teilen Calciumbromid und 4 bis 2000 Gew.-Teilen (eines) entzündungshemmende(n) Steroide (Steroids) enthält.

8. Verwendung nach Anspruch 6 oder Anspruch 7, wobei die Zusammensetzung eine Flüssigkeit mit einem pH-Wert im Bereich von 4 bis 7 ist.

9. Verwendung nach Anspruch 6, wobei die Injektion einmal oder zweimal pro Woche verabreicht wird.

10. Verwendung nach Anspruch 6, wobei die Injektion ein- bis viermal pro Woche verabreicht wird.

11. Verwendung nach Anspruch 9 oder Anspruch 10, wobei die Zusammensetzung 0,1 bis 25 mg Dibucainhydrochlorid, 0,1 bis 250 mg des pharmazeutisch annehmbaren Salzes von Salicylsäure, 0,05 bis 125 mg Calciumbromid und 0,004 bis 500 mg des entzündungshemmenden Steroids pro Dosis enthält.

12. Zusammensetzung zur Verstärkung der schmerzlindernden Wirkung einer Dibucain, ein pharmazeutisch akzeptables Salz von Salicylsäure und Calciumbromid enthaltenden Zusammensetzung, die als aktiven Bestandteil ein entzündungshemmendes Steroid enthält.

13. Zusammensetzung nach Anspruch 12, die zur Verstärkung der schmerzlindernden Wirkung einer Zusammensetzung dient, die 100 Gew.-Teile Dibucain, 100 bis 1000 Gew.-Teile eines pharmazeutisch annehmbaren Salzes von Salicylsäure und 50 bis 500 Gew.-Teile Calciumbromid enthält.

14. Zusammensetzung zur Verstärkung der schmerzlindernden Wirkung nach Anspruch 12 oder Anspruch 13, wobei das entzündungshemmende Steroid wenigstens eine Element ist, das aus der aus Dexamethason, Betamethason, Methylprednisolon, Prednisolon und deren pharmazeutisch annehmbaren Salzen bestehenden Gruppe ausgewählt ist.

15. Verwendung eines entzündungshemmenden Steroids für die Herstellung einer Zusammensetzung mit einer potenzierten schmerzlindernden Wirkung, die als aktiven Bestandteil das entzündungshemmende Steroid umfaßt, wobei die Zusammensetzung zusammen mit einer Zusammensetzung, die Dibucain, ein pharmazeutisch annehmbares Salz von Salicylsäure und Calciumbromid enthält, für die Behandlung von Schmerzen verwendet wird.

16. Verwendung nach Anspruch 15, wobei die Zusammensetzung mit einer potenzierten schmerzlindernden Wirkung verwendet wird, indem sie zu einer injizierbaren Zusammensetzung gegeben wird, die Dibucain, ein pharmazeutisch annehmbares Salz von Salicylsäure und Calciumbromid enthält.

17. Verwendung nach Anspruch 15, wobei die Zusammensetzung mit einer potenzierten schmerzlindernden Wirkung unabhängig von, aber zusammen mit einer Zusammensetzung verwendet wird, die Dibucain, ein pharmazeutisch annehmbares Salz von Salicylsäure und Calciumbromid enthält

18. Verwendung nach einem der Ansprüche 15 bis 17, wobei das entzündungshemmende Steroid wenigstens ein Element ist, das aus der aus Dexamethason, Betamethason, Methylprednisolon, Prednisolon und deren pharmazeutisch annehmbaren Salzen bestehenden Gruppe ausgewählt ist.

19. Verwendung nach einem der Ansprüche 15 bis 17, wobei das entzündungshemmende Steroid mit einem Anteil von 0,25 bis 800 Gew.-Teilen auf 100 Gew.-Teile der Gesamtmenge an Dibucain, einem pharmazeutisch annehmbaren Salz von Salicylsäure und Calciumbromid enthalten ist.

## Revendications

1. Composition injectable pour le traitement de la douleur, contenant de la dibucaïne, un sel pharmacologiquement acceptable de l'acide salicylique, du bromure de calcium et un ou des stéroïde(s) antiphlogistique(s).

2. Composition pour le traitement de la douleur selon la revendication 1, qui est un liquide ayant un pH compris entre 4 et 7.

3. Composition pour le traitement de la douleur selon la revendication 1 ou la revendication 2, contenant 100 parties en poids de dibucaïne, 100-1000 parties en poids d'un sel pharmacologiquement acceptable de l'acide salicylique, 50-500 parties en poids de bromure de calcium et 4-2000 parties en poids de stéroïde(s) antiphlogistique(s).

4. Composition pour le traitement de la douleur selon la revendication 1, contenant 0,1-25 mg d'hydrochlorure de dlbucaïne, 0,1-250 mg d'un sel pharmacologiquement acceptable de l'acide salicylique, 0,05-125 mg de bromure de calcium et 0,004-500 mg de stéroïde(s) antiphlogistique(s) par administration à un seul point d'injection.

5. Composition pour le traitement de la douleur selon l'une quelconque des revendications 1 à 4, dans laquelle le stéroïde antiphlogistique est au moins un élément choisi dans le groupe constitué par la dexaméthasone, la bétaméthasone, la méthylprednisolone, la prednisolone et leurs sels pharmacologiquement acceptables.

6. Utilisation d'une composition contenant de la dibucaïne, un sel pharmacologiquement acceptable de l'acide salicylique, du bromure de calcium et un stéroïde antiphlogistique, pour la préparation d'une composition injectable pour le traitement de la douleur.

7. Utilisation selon la revendication 6, dans laquelle la composition contient chaque élément dans une proportion de 100 parties en poids de dibucaïne, 100-1000 parties en poids d'un sel pharmacologiquement acceptable de l'acide salicylique, 50-500 parties en poids de bromure de calcium et 4-2000 parties en poids de stéroïde(s) antiphlogistique(s).

8. Utilisation selon la revendication 6 ou la revendication 7, dans laquelle la composition est un liquide ayant un pH compris entre 4 et 7.

9. Utilisation selon la revendication 6, dans laquelle la composition injectable est administrée une fois ou deux fois par semaine.

10. Utilisation selon la revendication 6, dans laquelle la composition injectable est administrée 1 à 4 fois par semaine.

11. Utilisation selon la revendication 9 ou la revendication 10, dans laquelle la composition contient 0,1-25 mg d'hydrochlorure de dibucaïne, 0,1-250 mg d'un sel pharmacologiquement acceptable de l'acide salicylique, 0,05-125 mg de bromure de calcium et 0,004-500 mg de stéroïde antiphlogistique par dose.

12. Composition destinée à renforcer l'action de traitement de la douleur d'une composition contenant de la dibucaïne, un sel pharmacologiquement acceptable de l'acide salicylique et du bromure de calcium, contenant un stéroïde antiphlogistique en tant que principe actif.

13. Composition selon la revendication 12, destinée à renforcer l'action de traitement de la douleur d'une composition contenant 100 parties en poids de dibucaïne, 100-1000 parties en poids d'un sel pharmacologiquement acceptable de l'acide salicylique et 50-500 parties en poids de bromure de calcium.

14. Composition destinée à renforcer l'action de traitement de la douleur selon la revendication 12 ou la revendication 13, dans laquelle le stéroïde antiphlogistique est au moins un élément choisi dans le groupe constitué par la dexaméthasone, la bétaméthasone, la méthylprednisolone, la prednisolone et leurs sels pharmacologiquement acceptables.

15. Utilisation d'un stéroïde antiphlogistique pour la préparation d'une composition ayant une action renforcée de traitement de la douleur, qui contient, en tant que principe actif, le stéroïde antiphlogistique, la composition étant utilisée pour le traitement de la douleur en association avec une composition contenant de la dibucaïne, un sel pharmacologiquement acceptable de l'acide salicylique et du bromure de calcium.

16. Utilisation selon la revendication 15, dans laquelle la composition ayant une action renforcée de traitement de la douleur est utilisée en l'ajoutant à une composition injectable contenant de la dibucaïne, un sel pharmacologiquement acceptable de l'acide salicylique et du bromure de calcium.

17. Utilisation selon la revendication 15, dans laquelle la composition ayant une action renforcée de traitement de la douleur est utilisée indépendamment mais en même temps qu'une composition contenant de la dibucaïne, un sel pharmacologiquement acceptable de l'acide salicylique et du bromure de calcium.

18. Utilisation selon l'une quelconque des revendications 15 à 17, dans laquelle le stéroïde antiphlogistique est au moins un élément choisi dans le groupe constitué par la dexaméthasone, la bétaméthasone, la méthylprednisolone, la prednisolone et leurs sels pharmacologiquement acceptables.

19. Utilisation selon l'une quelconque des revendications 15 à 17, dans laquelle le stéroïde antiphlogistique est contenu dans une proportion de 0,25-800 parties en poids pour 100 parties en poids de la teneur totale en dibucaïne, un sel pharmacologiquement acceptable de l'acide salicylique et du bromure de calcium.
